Europäisches Patentamt

European Patent Office

Office européen des brevets

(11) Publication number: **0 352 923**
**A1**

(12) **EUROPEAN PATENT APPLICATION**

(21) Application number: 89306879.1

(22) Date of filing: 06.07.89

(51) Int. Cl.⁴: **A61B 5/00 , G01N 21/31**

(30) Priority: 25.07.88 US 223592

(43) Date of publication of application:
31.01.90 Bulletin 90/05

(84) Designated Contracting States:
DE FR GB

(71) Applicant: **BAXTER INTERNATIONAL INC. (a Delaware corporation)**
**One Baxter Parkway**
**Deerfield Illinois 60015(US)**

(72) Inventor: **Cross, Klaus**
**1976 Dennison Street**
**Gloucester, MA 01930(US)**

(74) Representative: **MacGregor, Gordon et al**
**ERIC POTTER & CLARKSON 14 Oxford Street**
**Nottingham, NG1 5BP(GB)**

(54) Spectrophotometric apparatus and method for monitoring oxygen saturation.

(57) A continuous spectrum of light (10) in a selected wavelength (12) band is transmitted through selected tissue. The spectral distribution of light transmitted through the selected tissue is monitored and differentiated (36) with respect to wavelength. The differentiated spectral data is processed (38) to determine the oxygen saturation in the tissue. The spectral data may be digitized (34) and stored in a storage device (33) that correlates the transmitted intensity with the wavelength. The intensity values for a predetermined number of spectra may be averaged (35) to smooth the spectral data. The first derivative of the averaged spectral data is then determined at predetermined wavelengths and processed (38) with an algorithm to determine the oxygen saturation.

## SPECTROPHOTOMETRIC APPARATUS AND METHOD FOR MONITORING OXYGEN SATURATION

### BACKGROUND OF THE INVENTION

This invention relates generally to measurement of oxygen saturation in living tissue and particularly to non-invasive techniques for such measurements. Still more particularly, this invention relates to noninvasive spectrophotometric measurement of oxygen saturation of blood in body organs such as the brain.

Oxygen sufficiency and metabolism are parameters of fundamental importance in assessing the function of any body organ. In the absence of sufficient oxygen, provision of energy for tissue functions becomes impaired and is accompanied by a corresponding impairment in organ functioning. In case of extensive oxygen deprivation over a period of time, the organ looses viability, which may cause severe illness or even death.

Although all oxygen insufficiency adversely effects all body organs, the effect is most acute for the brain because of the brain's sensitivity with respect to oxygen demand and its dependance upon oxidative metabolism for proper functioning and viability. For example, it is known that hypoxia or anoxia of the brain for more than about a dozen seconds produces dysfunction, and an absence of oxygen for longer than a few minutes causes irreversible brain damage. A less acute impairment of oxygen availability causes a gradual loss in brain function, particularly in the higher centers of the cerebral cortex.

Because of the role that oxygen sufficiency plays in human physiology, intensive efforts have been made over the years to measure oxygenation in various organs. Brain and heart oxygenation are particularly important in this regard. However, it is difficult to obtain a direct measurements of oxygen saturation by a non-invasive technique except by means of pulse oximetry, which measures arterial oxygen saturation. Conway, *et al.* "A new approach for the estimation of body composition: infrared interactance.", Am. J. Clin. Nutr. 40: 1123-1130, 1984, reports that $O_2$ dependent organs such as the brain and heart regulate their $O_2$ delivery independently of changes in arterial $O_2$ content. Therefore, the effects of spontaneous changes in systemic $O_2$ availability for these organs cannot be measured by pulse oximetry.

Previous methods have used secondary effects, such as electroencephalographic and electrocardiographic changes to detect hypoxia. Electroencephalographic recordings indicating dysfunction are mainly useful for diagnosis of severe hypoxic or anoxic conditions in the brain. Electrocardiograph recordings are used to establish an oxygen deficiency in the heart muscle. However, these methods are diagnostic only in far advanced cases, and both the organs and the patient are in a precarious state of health before these signals become indicative of pathology.

It is commonly assumed that either insufficient blood circulation or ventilation is the main cause of inadequate oxygen delivery to cerebral and myocardial tissue. Although this assumption is probably correct in the majority of cases, blood flow measurements are indirect global measurements of organ oxygen delivery and are incapable of distinguishing adequate regional blood flow. Similarly, pulse oximetry is a global measurement of ventilation and oxygen delivery and is incapable of distinguishing regional ventilation and perfusion

It is known that the human body and its organs are relatively pervious to low level, non-hazardous light energy in the near infrared region of the spectrum. A beam of relatively low intensity radiation in the wavelength region of about 700 to 900 nm can penetrate and propagate through a relatively long optical path in any selected portion of a human or animal body that includes both bone and soft tissue. In particular, low intensity radiation in the 700 to 900 nm wavelength range will penetrate and propagate through a subject's skull and brain tissue with an intensity sufficient for detection with a conventional photodetector. Both hemoglobin and oxyhemoglobin absorb light in the 700 to 900 nm wavelength range. Therefore, the spectrum of such light transmitted through a subject's head may be analyzed to determine the level of oxygenation in brain tissue.

Considerable work has been done to develop techniques for non-invasively and atraumatically measuring blood oxygenation in particular, optical measurement techniques have been the subject of extensive research. The patents and publications discussed subsequently are representative of the prior art.

U.S. Patents 3,994,592 to Lardon *et al.*; 4,273,442 to Lubbers; 4,086,915 to Kofsky *et al.* and RE 31,879 to Lubbers *et al.* disclose spectral photometry apparatus that may be used for hemoglobin oxygenation testing. All four of these patents discuss or disclose differentiation of the signal at various wavelengths with respect to time. However, none of these patents discloses differentiation with respect to wavelength

U.S. Patent 3,994,592 to Lardon *et al.* is directed to a method for determining the ratio of the concentration of two substances in a mixture of substances having adjacent absorption bands such that the radiation absorption of the mixture has a minimum between the absorption bands. Measuring absorption in three narrow spectral regions of the radiation transmitted through the mixture determines the concentration. The differences between the transmitted intensities in the three bands are used to determine the concentration ratio.

U.S. Patent 4,086,915 to Kofsky et al. is directed to an ear oximetry process and apparatus that uses light of two or more discrete wavelengths. Kofsky directs light of a first wavelength on an area of the subject's skin and detects the transmitted intensity. Light of a second wavelength is then directed upon the same area of the subject's skin and the transmitted intensity is again measured. Electrical signals indicative of the transmitted light intensities at the two wavelengths are time differentiated. The differentiated signals are processed with a set of predetermined coefficients to obtain the arterial oxygen saturation of the blood.

U.S. Patent 4,273,442 to Lubbers is concerned with the display of hemoglobin spectra derived from use of a spectrophotometer on the surface of body tissue. Lubbers discloses a spectral photometer that emits light toward a subject and detects the light reflected back from the subject. Successive maxima of the spectral distribution are detected, and the distances between successive maxima are calculated.

U.S. Patents 4,281,645 and 4,223,680 to Jobsis are directed to a spectrophotometric method and apparatus for making non-invasive n vivo measurements of local metabolism of a body organ such as the brain. Jobsis discloses selecting an optical path intersecting the organ and extending for several centimeters between points of light entry and exit on the surface of the body. Jobsis also discloses placing a plurality of near infrared light sources outside the body to provide light emissions of different wavelength in the 700 to 1300 nanometer spectral range. The light emissions have intensities below the level damaging to the body and the organ but sufficient to be detectable by a light sensor after transmission along the optical path. Jobsis discloses optical emissions including at least one measuring wavelength and at least one reference wavelength within the spectral range. Each measuring wavelength is selected such that the organ n vivo exhibits a selective absorption. The absorption depends upon a specific state of metabolic activity of the organ n vivo.

Both of these two Jobsis patents disclose directing measuring and reference wavelengths sequentially along the path and through the organ and receiving the transmitted light emissions at a light sensor. An electric circuit means produces an electrical signal representing the difference in absorption of the measuring and reference wavelengths by the organ as a function of the state of the metabolic activity n vivo The electrical signals are indicative of the particular state of metabolic activity of the organ. The method disclosed by Jobsis for determining qualitative changes in oxygen concentration depends upon the light absorbing characteristics of the enzyme cytochrome a, $a_3$, which is also known as cytochrome c oxidase.

The key element of Jobsis is the measurement of qualitative changes in oxygen concentration. Probes are placed on a subject's head to establish a baseline or reference point. Changes from the baseline are not calibrated.

U.S.Patent 4,510,938 to Jobsis relates to techniques for directing light to the head of a subject.

Stauffer et al., Derivative Spectroscopy, Applied Optics, Vol. 7, No. 1, pp. 61-65, January, 1968 discloses that derivative spectroscopy has been used to enhance the detectability of overlapping spectral bands. The paper points out that derivative spectroscopy suppresses background radiation, reduces effects of instrumentally scattered radiation and suppresses atmospheric turbulence effects.

Ewalt et al., Derivative Spectrometer, IBM Technical Disclosure Bulletin, Vol. 20, No. 3, pp. 1137-1140, August 1977 discloses that measuring the derivative with respect to wavelength of the usual transmission curve can improve the detection limit by an order of magnitude. This paper states that the derivative output signal is directly proportional to the concentration of absorbers in the sample path. The paper discusses a derivative spectrometer that uses a repetitive scan of a narrow wavelength band centered on the line or band peak of interest.

U.S.Patent 4,463,762 to Rubens discloses a technique for measuring oxygen absorption. Rubens uses a tourniquet to form a region of occlusion. Light of two different wavelengths is directed onto the skin tissue of the subject, and the amount of each wavelength absorbed is measured. Rubens determines the rate of change of the absorption of one wavelength relative to the other for producing a signal that indicates the rate of oxygen utilization in the tissue.

U.S.Patent 4,213,462 to Sato is directed to the detection of abnormalities such as cancerous growths. Tissue being tested is subjected to a first predetermined pressure and then exposed to light. The radiation reflected or absorbed is measured, and then the tissue is subjected to a second predetermined pressure and again exposed to the light. The difference in the absorption or reflectance characteristics for the different pressures indicates whether the tissue contains an abnormality.

U.S.Patent 4,548,505 to Ono is directed to a sensor for a spectral analyzer. The purpose of the Ono patent is to provide means for obtaining data as soon as the tip of the bundle of fibers contacts the tissue and before any change occurs in the tissue. A second purpose of this invention is to provide constant pressure on the tissue when data is taken to maintain a local hemostatic effect.

U.S.Patent 4,017,192 to Rosenthal relates to a technique for optical analysis of biomedical specimens where three interference filters are used to selectively pass light of a particular frequency through a biological sample. Rosenthal is particularly directed to automatic detection of abnormalities, such as cancer. Transmittance or reflectance data over a large number of wavelengths are correlated by multiple linear regression analysis with conventional clinical results to select test wavelengths and constants for a correlation equation. The correlation equation is applied to the spectral data to determine quantitatively the presence of the abnormality.

## SUMMARY OF THE INVENTION

The present invention provides an improved apparatus and method for non-invasively and atraumatically quantitatively determining the oxygen saturation in living tissue. This invention has the advantages of using only a single light source and permitting continuous measurements of the spectrum transmitted through the tissue of interest.

An apparatus for measuring oxygen saturation in living tissue according to the present invention comprises means for directing a continuous spectrum of light in a selected wavelength band through selected tissue, means for determining the spectral distribution of light transmitted through the selected tissue, means for determining the first derivative of the spectral distribution of the light transmitted through the selected tissue with respect to wavelength, and means for processing the first derivative to determine the oxygen saturation in the tissue.

The means for determining the spectral distribution of light transmitted through the tissue preferably includes light detecting means for producing signals indicative of the light intensity transmitted through the tissue, and means for correlating the transmitted intensity with the wavelength of light incident on the tissue.

The apparatus according to the present invention may further include means for digitizing the signal output from the light detecting·means, storage means for storing digitized intensity data as a function of wavelength, and means for averaging the intensity values for a predetermined number of spectra. The differentiating means is preferably configured to determine the first derivative of the averaged spectral data at predetermined wavelengths. The differentiating means is preferably configured to determine the oxygen saturation in the tissue by calculating a linear combination of the first derivatives of the spectral data at predetermined wavelengths.

A method according to the present invention for measuring oxygen saturation in living tissue, comprises the steps of directing a continuous spectrum of light in a selected wavelength band through selected tissue, determining the spectral distribution of light transmitted through the selected tissue, determining the first derivative of the spectral distribution of the light transmitted through the selected tissue with respect to wavelength, and processing the first derivative to determine the oxygen saturation in the tissue.

The step of determining the spectral distribution of light transmitted through the tissue preferably includes the steps of producing signals indicative of the light intensity transmitted through the tissue and correlating the transmitted intensity with the wavelength of light incident on the tissue.

The method according to the present invention also preferably includes the steps of digitizing the signal output from the light detecting means; storing digitized intensity data as a function of wavelength, and averaging the intensity values for a predetermined number of spectra. The method may also further include the step of forming the differentiating means to determine the first derivative of the averaged spectral data at predetermined wavelengths. The method may include the step of calculating the oxygen saturation using the algorithm

$$[O_2]_{SAT} = A\frac{\partial I_a}{\partial \lambda} + B\frac{\partial I_b}{\partial \lambda} + C\frac{\partial I_c}{\partial \lambda} + D$$

$$= A\frac{\partial I_a/\partial \lambda}{I_b/\partial \lambda} + B\frac{\partial I_c}{\partial \lambda} + C$$

where A, B, C and D are constants; a, b and c represent selected points in the transmitted spectrum and λ the wavelength. Another algorithm that may be used in the method of the invention is where the constants have been previously defined.

## BRIEF DESCRIPTION OF THE DRAWINGS

Figure 1 is a block diagram of the apparatus of the invention;

Figure 2 illustrates a filter structure that may be used to obtain a continuous spectrum in a selected wavelength range;

Figure 3 graphically illustrates the logarithm of the reciprocal of spectral data obtained using the apparatus and methods of the present invention; and

Figure 4 graphically illustrates the first derivative of the logarithm of the reciprocal of the spectral data of Figure 3.

## DESCRIPTION OF THE PREFERED EMBODIMENT

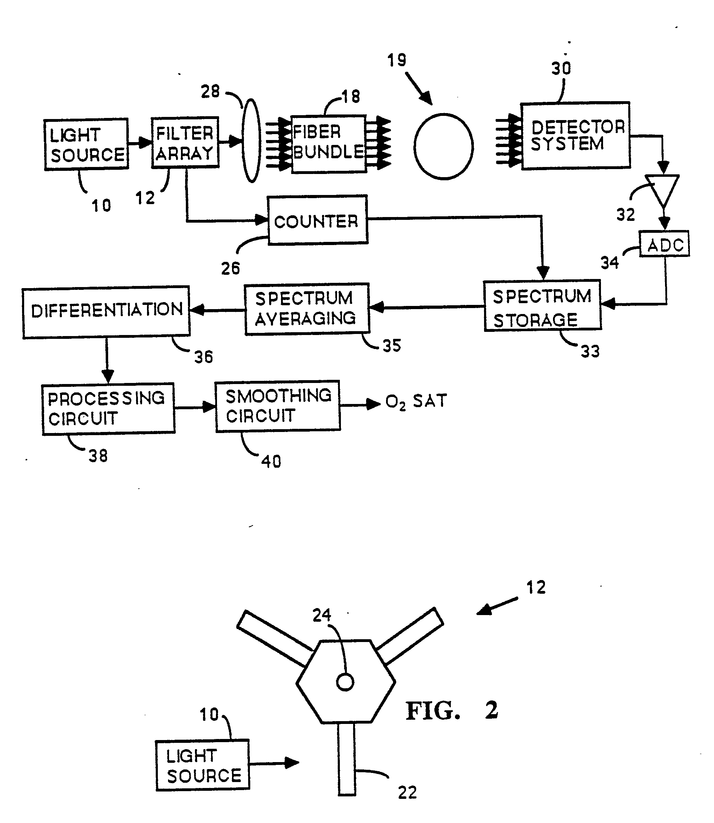

Referring to the block diagram of Figure 1, a light source 10 provides light to an optical filter assembly 12. The light source may be either a white light source or an infrared lamp that produces wideband light in a selected wavelength range. The wavelength range preferably includes wavelengths of about 700 to 900 nm.

A filter assembly suitable for use in the present invention is described in U.S Patent 4,017,192 to Rosenthal, which is hereby incorporated by reference into this disclosure. Referring to Figure 2, the filter assembly 12 is preferably in the form of a paddle wheel 16 mounted for rotation about an axis perpendicular to the light path between the lamp 10 and a fiber optic bundle 18. The paddle wheel 16 is typically driven at about 3 revolutions per second. In a preferred embodiment, three narrow bandwidth plate-shaped optical interference filters 22 are mounted on a hexagonal axle 24. The planes of the filters 22 are equally separated by angles of about 120°. The filters 22 extend radially from the axle 24 in planes that intersect approximately in a line coincident with the axis of rotation of the paddle wheel 16. The filters 22 preferably extend equal radial distances from the axis. The cross sectional configuration of the filters 22 on the axle 24 thus resembles the letter Y. This arrangement of a single light source and three rotating filters 22 provides a continuous spectral range in the near infrared region of the spectrum between the wavelengths of about 700 nm and 1000 nm.

Referring to Figures 1 and 2, a signal indicative of the angle of rotation of the filter assembly 12 is passed through a digital counter circuit 26 that is set to provide control signals corresponding to selected angles of rotation of the paddle wheel 16. Since the angle of rotation is related to the wavelength output of the filter assembly 12, the control signals also indicate the wavelength of light that is being applied to the subject. A counter circuit suitable for use in the present invention is disclosed in U.S. Patent No. 3,861,788 to Webster, the disclosure of which is hereby incorporated by reference into the present disclosure. An optical interrupter may be used to correlate the wavelength and intensity so that spectral data comprising intensity as a function of wavelength may be measured.

The light beam output from the filter assembly 12 may then impinge upon a lens system 28 that focuses the light onto the fiber optic bundle 18. The fiber optic bundle 18 is convenient for directing the light to a portion of a subject's body such as the head, where tissue hemoglobin oxygenation is to be measured. The lens system 28 preferably is designed to provide a wide beam of essentially uniform intensity over the fiber bundle 18. The fiber optic bundle 18 is preferably an assembly of multimode optical fibers (not shown) having a numerical aperture suitable for receiving light input from the lens system 28. Such fiber cptic bundles are well-known in the art and are therefore not described in detail herein. The light is typically input to the subject's forehead or to the top of the head. Applying the light to the top of the subject's head permits direct measurements of hemoglobin saturation in the sagittal sinus, which is where venous blood pools in the top of a person's head.

A detector system 30 that includes at least one photodetector (not shown) detects the waves output from the tissue and forms an electrical signal indicative of the detected light intensity. When the detector system includes a single photodetector, the output of the detector system 30 is amplified by an amplifier 32, which may be a logarithmic amplifier if the range of the electrical output of the detector is sufficiently broad. The analog output of the amplifier 32 is then converted to a digital signal by an analog to digital converter (ADC) 34.

More than one detector may be used to determine the saturation in various parts of the head. If multiple detectors are used then, there will be an amplifier (not shown) and an ADC (not shown) for each detector. For example, a detector may be placed within one to two centimeters of the light source for measuring the saturation of the skull and skin. A second detector may be used to measure the far field at distances greater than three centimeters from the source. This far field detector may be used to measure the average saturation of the skin, skull and brain. The spectral difference between the far field and near field detectors then are indicative of the brain saturation.

The output of the detector 30 is sampled at enough wavelengths to obtain data that can be processed to obtain at least a first derivative of the absorption of oxyhemoglobin with respect to wavelength. A spectrum storage device 33 stores the sampled detector output. The counter circuit 26 has its control signal output connected to the storage device 32 to correlate the detected and stored intensity data with the wavelength incident on the tissue. A spectrum averaging circuit 35 averages the spectral data and provides the averaged output to a differentiator 36. A signal processing device 40 then applies an algorithm to the smoothed derivative to determine the $O_2$ saturation in the tissue.

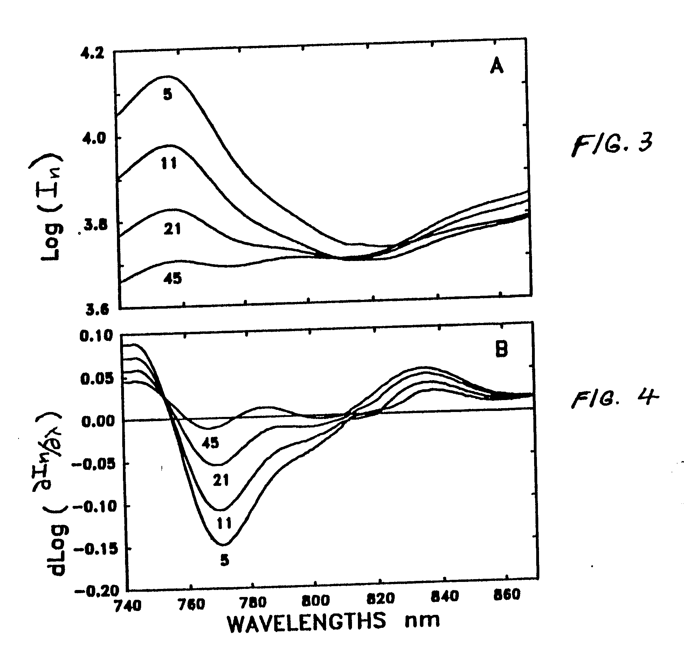

The spectral data from about 700 nm to 1000 nm is divided and stored in 300 wavelength intervals of unequal spectral bandwidth. In order o enhance the signal to noise ratio, a number of spectra may be stored and time averaged to produce the spectra shown in Figure 3. For example, the light source and associated electronics may be used to obtain three spectra per second. Each measured spectrum is stored in the storage device 33. Spectrum averaging circuit 34 averages a selected number of spectra. Figure 3 is a plot of the logarithm of the reciprocal of the intensity for 100 averaged spectra.

The first derivative of the spectral data is preferably calculated at 766, 835 and 865 nm although other wavelengths in the 700 to 1000 nm range have been shown to give similar results. In order to enhance the spectral features of interest, the calculation of the derivative preferably involves an averaging process rather than merely subtracting successive intensity values and dividing by the change in wavelength. For example, the spectral data may be divided into 300 intensity intervals or segments. In order to find the derivative of the thirteenth segment, the average value $(I_{1-10})$ for the first ten segments may be calculated and stored. The next five segments $(I_{11-15})$ may then be skipped. The average intensity for the next ten segments 16-25 $(I_{16-25})$ is then calculated. The derivative is then found by dividing the difference of the intensity averages by the change in wavelength. The number of segments averaged is referred to as the segment size and the number of segments skipped is referred to as the gap. Figure 4 shows the derivative of the spectral data of Figure 3 for a segment size of 10 and a gap of 1.

A preferred algorithm for calculating the oxygen saturation uses a constant plus a linear combination of the first derivatives of the spectral data. For example the oxygen saturation may be found from the thirteenth, twenty-seventh and ninetieth data points so that

$$[O_2]_{SAT} = A\frac{\partial I_{13}}{\partial \lambda} + B\frac{\partial I_{27}}{\partial \lambda} + C\frac{\partial I_{90}}{\partial \lambda} + D.$$

The oxygen saturation may also be written as

$$[O_2]_{SAT} = A\frac{\partial I_a/\partial \lambda}{\partial I_b/\partial \lambda} + B\,\partial I_c/\partial \lambda + C.$$

Referring to Figures 3 and 4, one set of preferred data points and the corresponding wavelengths are: segment 30 = 766 nm; segment 164 = 835 nm and segment 235 = 865 nm. Therefore the oxygen saturation is

$$[O_2]_{SAT} = A\frac{\partial I_{30}/\partial \lambda}{\partial I_{164}/\partial \lambda} + B\,\partial I_{235}/\partial \lambda + C.$$

The constants are determined in the calibration of each instrument. For one instrument that has been used A = 4.046; B = 519.546 and C = 26.488. Other algorithms may be used to calculate the oxygen

6

saturation. In some cases it may be desirable to use a plurality of different algorithms to obtain the saturation. The saturations may then be averaged. If there is reason to believe that one algorithm may be more reliable than another, appropriate weighting factors may be used in the averaging process.

The derivative of the spectral data may be smoothed before processing by a CPU 38 with an algorithm that produces a quantitative measurement of the hemoglobin oxygenation in the tissue. The smoothing circuitry may include digital filter circuitry for calculating a weighted average of the previous signal and the new signal. For example, the output of the smoothing circuit may be 90% of the previous value plus 10% of the new value. The saturation output may be expressed as

$$SAT_{OUT} = 0.1 \; SAT_{CUR} + 0.9 SAT_{PREV}.$$

Both methods of smoothing prevent the signal input to the processor from changing too rapidly.

The method of the invention may also include means for calculating the second and higher derivatives of the spectral data curve with respect to wavelength. Using the derivatives of the spectral data to calculate the oxygen saturation eliminates errors caused by nonspecific absorptions and light scattering by the skull, scalp and dura. The second derivative may be obtained numerically from the first derivative in the same manner that the first derivative is obtained from the spectral data.

The apparatus of the present invention may be used to make a tomographic map of a selected portion of a subject's body such as the brain or heart. Localized changes is cellular oxidative metabolism are indicative of conditions such as stroke, infarction, oligemia and ischemia. The transmitted spectrum is processed as described above for light paths in different planes through the organ of interest. The data for making a tomographic map may be obtained by moving the fiber optic bundle and the detector relative to the organ. The use of more than one detector according to the invention at a time may permit the data to be obtained in less time than would be required with the use of a single source and detector.

## Claims

1. Apparatus for measuring oxygen saturation in living tissue, comprising:
means for directing a continuous spectrum of light in a selected wavelength band through selected tissue;
means for determining the spectral distribution of light transmitted through the selected tissue;
means for determining a derivative with respect to wavelength of the spectral distribution of the light transmitted through the selected tissue; and
means for processing the derivative to determine the oxygen saturation in the tissue.

2. The apparatus of claim 1, wherein the means for determining the spectral distribution of light transmitted through the tissue includes: light detecting means for producing signals indicative of the light intensity transmitted through the tissue; and
means for correlating the transmitted intensity with the wavelength of light incident on the tissue.

3. The apparatus of claim 2, further including:
means for digitizing the signal output from the light detecting means;
storage means for storing digitized intensity data as a function of wavelength; and
means for averaging the intensity values for a predetermined number of spectra.

4. The apparatus of claim 3 wherein the differentiating means is configured to determine the first derivative of the averaged spectral data at predetermined wavelengths.

5. The apparatus of claim 4, further including means for determining the oxygen saturation in the tissue by calculating a linear combination of the first derivatives of the spectral data at the predetermined wavelengths.

6. The apparatus of claim 1 wherein the continuous spectrum includes wavelengths ranging from 700 nm to 1000 nm.

7. The apparatus of claim 2, further including:
a first photodetector for detecting signals indicative of the oxygen saturation of the skin and bones adjacent the tissue;
a second photodetector for detecting signals indicative of the oxygen saturation of the tissue and the skin and bones adjacent the tissue; and
means for processing signals output from the first and second photodetectors to determine the oxygen saturation of the tissue.

8. A method for measuring oxygen saturation in living tissue, comprising the steps of:
directing a continuous spectrum of light in a selected wavelength band through selected tissue;
determining the spectral distribution of light transmitted through the selected tissue;
determining the first derivative of the spectral distribution of the light transmitted through the selected tissue

7

with respect to wavelength; and
processing the first derivative to determine the oxygen saturation in the tissue.

9. The apparatus of claim 5, wherein, the means for determining oxygen saturation uses the formula

$$[O_2]_{SAT} = A \frac{\partial I_a}{\partial \lambda} + B \frac{\partial I_b}{\partial \lambda} + C \frac{\partial I_c}{\partial \lambda} + D$$

where A, B, C and D are constants; a, b and c represent selected points in the transmitted spectrum and $\lambda$ is the wavelength.

10. The apparatus of claim 9 wherein

$$[O_2]_{SAT} = A \frac{\partial I_a}{\partial \lambda} + B \frac{\partial I_b}{\partial \lambda} + C \frac{\partial I_c}{\partial \lambda}$$

$$+ D = = A \frac{\partial I_a / \partial \lambda}{\partial I_b / \partial \lambda} + B \, \partial I_c / \partial \lambda + C.$$

11. The apparatus of claim 10 wherein

$$[O_2]_{SAT} = A \frac{\partial I_{766} / \partial \lambda}{\partial I_{835} / \partial \lambda} + B$$

where $I_{766}$ is the intensity at a wavelength of 766 nm, $I_{835}$ is the intensity at a wavelength of 835 nm and $I_{865}$ is the intensity at a wavelength of 865 nm.

FIG. 2

FIG. 3

FIG. 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 046 601 (HELLIGE GMBH) * abstract; page 9, line 21 - page 14, line 13; figure 1 * | 1 | A 61 B 5/00 G 01 N 21/31 |
| A | | 2,4,5,8,9 | |
| Y | EP-A-0 063 431 (M. KONOMI) * abstract; page 9, line 18 - page 21, line 7, figures 4,13 * | 1 | |
| A | | 2,3,6 | |
| A,D | US-A-4 086 915 (H. I. KOFSKY et al.) * abstract; column 3, line 57 - column 4, line 25; figure 1 * | 1-5 | |
| A,D | IBM TECHNICAL DISCLOSURE BULLETIN vol. 20, no. 3, August 1977, pages 1137-1140, New York, USA; A. B. EWALT et al.: "Derivative Spectrometer" * whole document * | 1-3 | |

TECHNICAL FIELDS SEARCHED (Int. Cl.5)

A 61 B 5/00
A 61 B 5/14
G 01 N 21/31

The present search report has been drawn up for all claims

| Place of search | Date of completion of the search | Examiner |
|---|---|---|
| BERLIN | 17-10-1989 | WEIHS J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)